# EUROPÄISCHE PATENTANMELDUNG

(11) **EP 1 714 613 A1**
(43) Veröffentlichungstag der Anmeldung: **25.10.2006**
(21) Anmeldenummer: 05008933.3
(22) Anmeldetag: 22.04.2005
(51) Int. Cl.: A61B 5/15

(54) **Analytisches Hilfsmittel**

(71) Anmelder: F. HOFFMANN-LA ROCHE AG, 4070 Basel (CH); Roche Diagnostics GmbH, 68305 Mannheim (DE)
(72) Erfinder: Harttig, Herbert, 67434 Neustadt (DE)
(74) Vertreter: Isenbruck, Günter

(57) **Zusammenfassung**

Die Erfindung bezieht sich auf ein analytisches Hilfsmittel enthaltend einen Grundkörper (2), eine Lanzette (6) und ein Testelement (8), wobei
i) der Grundkörper (2) zwei über eine scharnierartige Verbindung (3) miteinander verbundene Teilkörper (4, 5) umfasst, wobei ein erster Teilkörper (4) die eine Lanzettenspitze (7) aufweisende Lanzette (6) und ein zweiter Teilkörper (5) das ein Testfeld (17) enthaltende Testelement (8) und einen Probenaufnahmeort (9) trägt und
ii) die zwei Teilkörper (4, 5) im Wesentlichen in einer gemeinsamen Ebene angeordnet sind und die Lanzette (6) durch eine mit dem ersten Teilkörper (4) verbundene Versiegelung (10) geschützt und von dem Testfeld (17) des Testelements (8) getrennt ist, so lange sich das analytische Hilfsmittel (1) in einem unbenutzten Zustand befindet, wobei die zwei Teilkörper (4, 5) um die scharnierartige Verbindung (3) aus der Ebene gegeneinander verschwenkbar sind und die Versiegelung (10) beim auftrennbar ist, so dass die Lanzettenspitze (7) zur Benutzung freigegeben wird.

## Beschreibung

Die Erfindung betrifft ein analytisches Hilfsmittel, das eine Lanzette und ein Testelement enthält, ein Verfahren zur Herstellung eines solchen analytischen Hilfsmittels und ein Verfahren zur Analyse einer Probe mit Hilfe eines solchen analytischen Hilfsmittels.

Die Untersuchung von Blutproben oder von interstitieller Flüssigkeit ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung der Probe kann die Haut zum Beispiel an der Fingerbeere oder dem Ohrläppchen der zu untersuchenden Person mit Hilfe einer sterilen, scharfen Lanzette perforiert werden, um so eine geringe Menge Blut oder interstitielle Flüssigkeit für die Analyse zu gewinnen. Insbesondere eignet sich diese Methode für die Analyse einer Probe, die unmittelbar nach der Probengewinnung durchgeführt wird.

Vor allem im Bereich des sogenannten "home-monitoring", also dort, wo medizinische Laien selbst einfache Analysen des Bluts oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (sogenannte Stechhilfen) angeboten, die eine möglichst schmerzarme und reproduzierbare Probengewinnung ermöglichen. Solche Lanzetten und Geräte (Stechhilfen) sind zum Beispiel Gegenstand von WO-A 98/48695, EP-A 0 656 970, US 4,442,836 oder US 5,554,166.

Die eigenhändige Blutzuckerbestimmung ist heute eine weltweit verbreitete Methode in der Diabetes-Kontrolle. Blutzuckergeräte im Stand der Technik bestehen aus einem Analysegerät, in das ein Testelement (Teststreifen) eingeführt wird. Das Testelement wird mit einem Tropfen einer Probe in Kontakt gebracht, der zuvor mittels einer Stechhilfe zum Beispiel aus der Fingerbeere gewonnen wurde. Analytische Testelemente dieser Art sind beispielsweise aus der CA 2,311,496 bekannt. Das dort beschriebene analytische Testelement enthält ein Nachweiselement und einen zum kapillaren Flüssigkeitstransport befähigten Kanal, wobei durch Kontakt des Testelements mit einem Blutstropfen an der Probenaufgabestelle Blut in Kontakt mit dem Nachweiselement gebracht wird und eine detektierbare Reaktion mit dem Analyten erfolgt.

Die zahkeichen, für die eigenhändige Blutzuckerbestimmung benötigten Systemkomponenten (Lanzette, Stechhilfe, Testelement und Analysegerät) benötigen viel Platz und bedingen eine relativ komplexe Handhabung. Inzwischen gibt es auch Systeme mit einem höheren Grad der Integration und damit einer einfacheren Handhabung, bei denen zum Beispiel die Testelemente im Analysegerät magaziniert und für die Messung zur Verfügung gestellt werden. Ein nächster Schritt der Miniaturisierung ist beispielsweise durch die Integration von mehreren Funktionen beziehungsweise Funktionselementen in einem einzigen analytischen Hilfsmittel (disposable) zu erreichen. Durch geeignete Kombination von Stechvorgang und sensorischer Analytkonzentrationserfassung auf einem Testelement lässt sich beispielsweise der Bedienablauf deutlich vereinfachen.

US 2003/0050573 A1 hat ein analytisches Hilfsmittel mit Lanzette und Testelement zum Gegenstand. Die Lanzette umfasst eine Lanzettennadel mit einer Spitze und einen Lanzettenkörper, der die Lanzettennadel zumindest im Bereich der Spitze vollständig umgibt, wobei die Lanzettennadel relativ zum Lanzettenkörper verschiebbar ist. Der Lanzettenkörper besteht zumindest im Bereich der Spitze der Lanzettennadel aus einem elastischen Material, in das die Spitze der Lanzettennadel eingebettet ist. Das analytische Testelement ist fest mit dem Lanzettenkörper verbunden.

US 2003/0,211,619 A1 bezieht sich auf Bänder mit darauf angeordneten Testern, die jeweils einen Teststreifensensor und eine Mikronadel umfassen. Das proximale Ende jedes Testers ist an dem Band befestigt, so dass der Tester, wenn er nicht versiegelt ist, von dem Rand wegbiegbar ist.

US 4,648,408 betrifft eine Blutuntersuchungsvorrichtung, die aus drei zusammenklappbaren Kunststofiblöcken aufgebaut ist, auf denen neben einer Nadel ein Reagenzstreifen und ein Desinfektionsmittel angeordnet sind.

US 2002/0052618 A1 hat ein analytisches Hilfsmittel mit integrierter Lanzette in einem Lanzettenkörper zum Gegenstand, wobei der Lanzettenkörper beweglich, zum Beispiel klappbar oder schwenkbar mit einem Rahmenelement eines Testelements verbunden ist, so dass die Lanzette einer Aufbewahrungsposition und eine Stechposition einnehmen kann, wobei die Lanzette in der Aufbewahrungsposition im Wesentlichen parallel zur Ebene des Testelements und in der Stechposition im Wesentlichen orthogonal zur Ebene des Testelements orientiert ist.

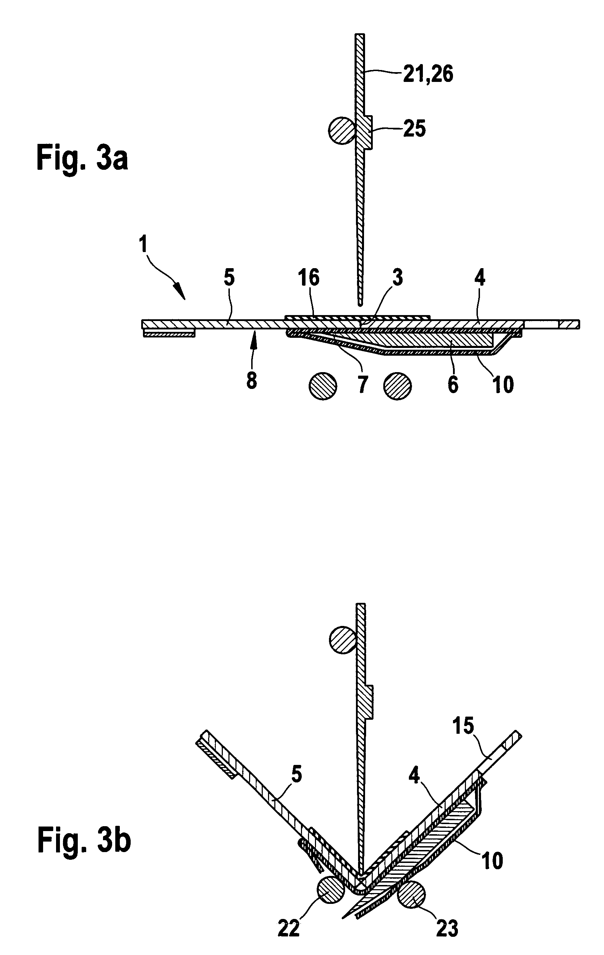

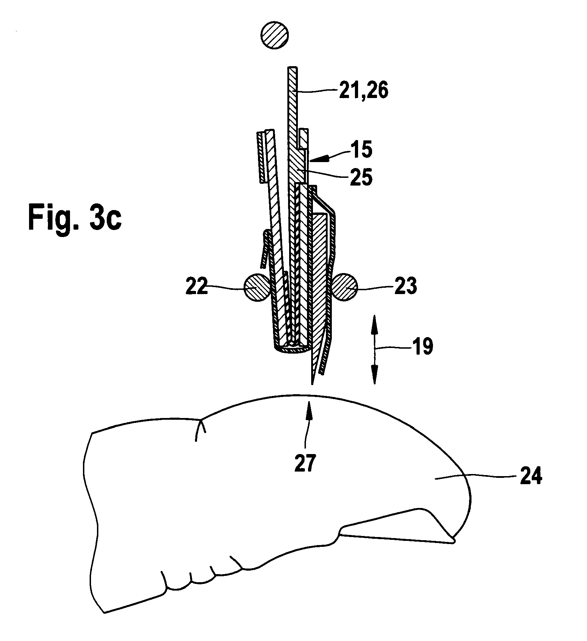

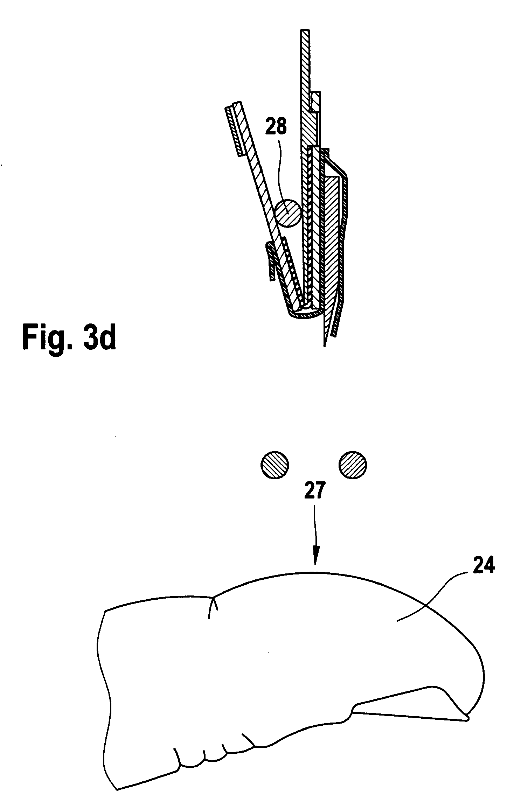

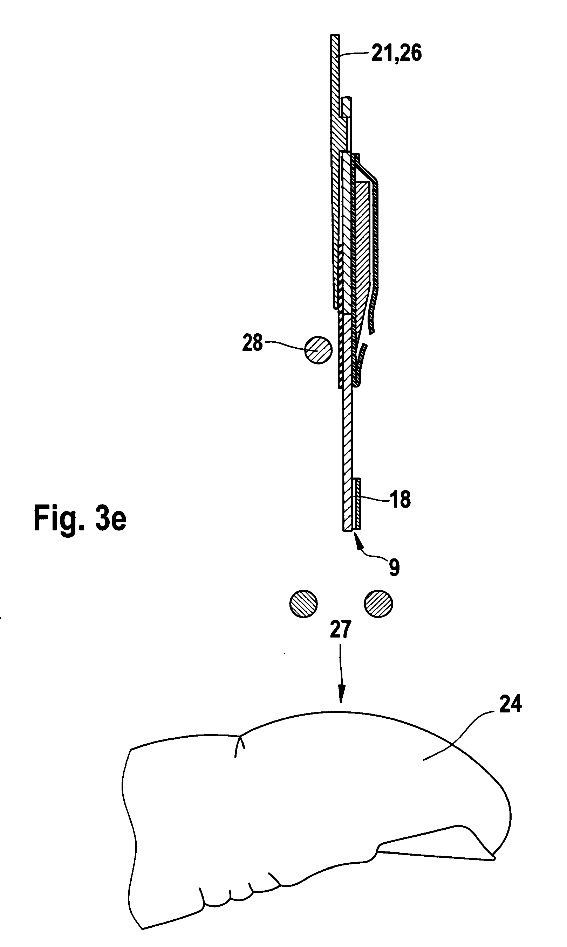

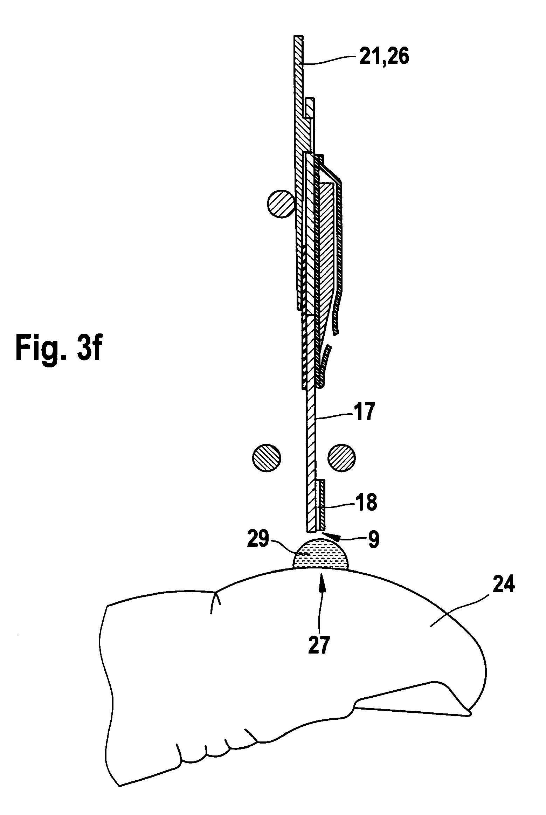

US 2004/0,064,068 A1 bezieht sich auf eine in einen Messstreifen integrierte Lanzettenanordnung. Gemäß einer beschriebenen Ausführungsform (Figur 3) ist der Winkel zwischen der Bewegung der Lanzette und der Ebene der Messoberfläche des Messstreifens variabel.

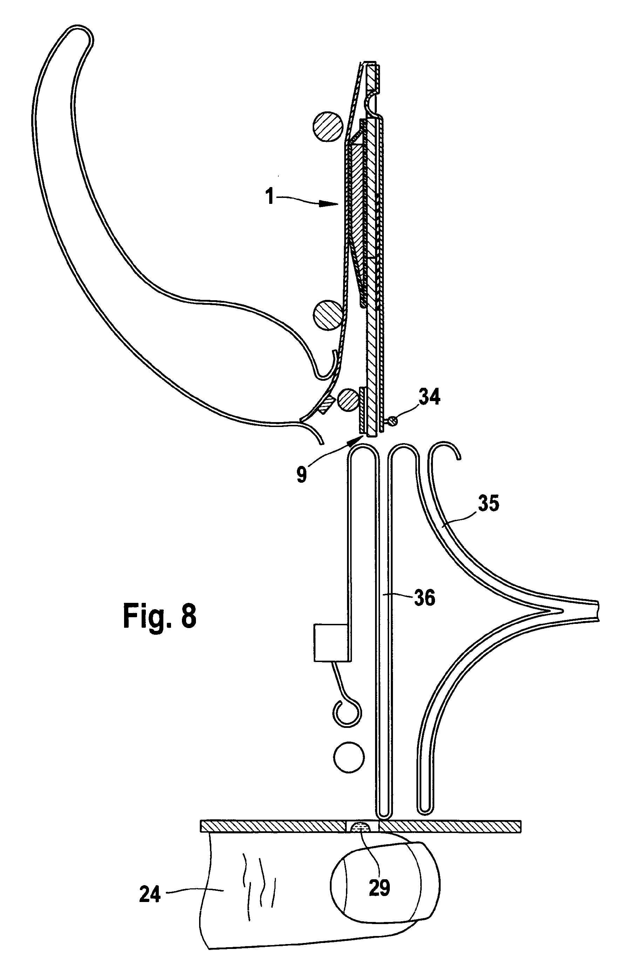

US 2003/0,212,347 A1 betrifft Vorrichtungen, die mindestens ein Hautperforationselement, einen Biosensor und einen Flüssigkeitstransportpfad zwischen dem Hautperforationselement und dem Biosensor aufweisen. Gemäß einer beschriebenen Ausführungsform (Figur 8) ist eine solche Vorrichtung so ausgebildet, dass das Hautperforationselement (Lanzette) aus der Ebene des Biosensors um eine Drehachse weggedreht werden kann.

EP 15 08 304 A1 bezieht sich auf eine medizinische Vorrichtung, die eine obere und eine untere flexible Lage, einen Lanzettenkörper und einen Teststreifen umfasst. Der Lanzettenkörper enthält eine Öffnung, in die sich ein Einstechelement erstreckt. Die Reaktionszone des Teststreifens steht mit der Öffnung in dem Lanzettenkörper in Kontakt. Dies hat den Nachteil, dass Bestandteile der auf der Reaktionszone vorhandenen Testchemie auf das Einstechelement gelangen können und dass ein Sterilisieren des Einstechelements nicht ohne ein unerwünschtes Sterilisieren der Reaktionszone möglich ist.

Diese im Stand der Technik bekannten analytischen Hilfsmittel sind kompliziert aufgebaut, großteils voluminös, unpraktisch in der Anwendung und kostspielig in der Produktion.

Die Herstellung von analytischen Hilfsmitteln birgt eine Vielzahl zu lösender Probleme. Die Lanzette ist zu sterilisieren und die Lanzettensterilität für den Zeitraum der Aufbrauchfrist des analytischen Hilfsmittels sicherzustellen. Die in dem Testfeld des Testelements vorhandene Testchemie kann durch die bekannten Verfahren zur Sterilisierung nachteilig in ihrer Funktions- und Wirkungsweise beeinflusst werden. Es können empfindliche chemische oder biologische Substanzen geschädigt werden. Die Testchemie sollte daher möglichst nicht dem Sterilisierungsverfahren für die Lanzette ausgesetzt werden. Für den Gebrauch des analytischen Hilfsmittels muss der Sterilschutz außerdem sicher, ohne Verletzungsgefahr für den Benutzer, entfernbar sein.

Die Aufgabe der vorliegenden Erfindung war es, die Nachteile des Standes der Technik zu vermeiden und die genannten Probleme zu lösen.

Diese Aufgabe wird erfindungsgemäß gelöst durch ein analytisches Hilfsmittel enthaltend einen Grundkörper, eine Lanzette und ein Testelement, wobei
i) der Grundkörper zwei über eine scharnierartige Verbindung miteinander verbundene Teilkörper umfasst, wobei ein erster Teilkörper die eine Lanzettenspitze aufweisende Lanzette und ein zweiter Teilkörper das ein Testfeld enthaltende Testelement und einen Probenaufnahmeort trägt und
ii) die zwei Teilkörper im Wesentlichen in einer gemeinsamen Ebene angeordnet sind und die Lanzette durch eine mit dem ersten Teilkörper verbundene Versiegelung geschützt und von dem Testfeld des Testelements getrennt ist, so lange sich das analytische Hilfsmittel in einem unbenutzten Zustand befindet, wobei die zwei Teilkörper um die scharnierartige Verbindung aus der Ebene gegeneinander verschwenkbar sind und die Versiegelung (z.B. beim Verschwenken der zwei Teilkörper) auftrennbar ist, so dass die Lanzettenspitze zur Benutzung freigegeben wird.

Bei diesem analytischen Hilfsmittel (oder synonym dazu "disposable") werden die drei Funktionen Einstechen, Probenaufnahme und Bereitstellen einer Testchemie zur Analyse der Probe zusammengefasst.

Der Grundkörper des analytischen Hilfsmittels dient als Träger für die Lanzette. Er umfasst zwei Teilkörper und ist vorzugsweise streifenförmig ausgebildet, wenn sich die zwei Teilkörper in einer Ebene befinden, also nicht gegeneinander verschwenkt sind. Als Abmessungen eines solchen streifenförmigen analytischen Hilfsmittels können beispielsweise eine Länge von ca. 38 mm und eine Breite von ca. 9 mm gewählt werden. An dem den Probenaufnahmeort aufweisenden Ende des zweiten Teilkörpers können die Ecken in einem 45°-Winkel um ca. 2 mm abgeschnitten werden. Eine gegebenenfalls vorhandene Abdeckung kann über diese abgeschnittenen Ecken hinausragen, so dass sie leicht greifbar ist.

Die Lanzette ist zum Perforieren der Haut eines Patienten an passenden Stellen mit der Lanzettenspitze zur Gewinnung einer Blutprobe oder einer Probe aus interstitieller Flüssigkeit vorgesehen. Als Lanzette kann eine runde, nadelförmige oder flache Lanzette dienen. Runde, nadelförmige Lanzetten werden üblicherweise aus Drahtabschnitten gefertigt, die durch Schleifen mit einer scharfen Spitze versehen sind. Flache Lanzetten werden üblicherweise durch Stanzen, Ätzen oder Laserschneiden aus Flachstahl gefertigt. Die Lanzette ist bei dem erfindungsgemäßen analytischen Hilfsmittel auf dem ersten Teilkörper befestigt. Die Lanzettenspitze ragt über den Rand des ersten Teilkörpers mindestens um die zur Perforation der Haut notwendige Einstechtiefe hinaus. Die Befestigung der Lanzette auf dem ersten Teilkörper erfolgt vorzugsweise durch Kleben, insbesondere mit Hilfe von Schmelzkleber oder durch Einschweißen oder -schmelzen. Der erste Teilkörper kann so vorstrukturiert sein, dass er Ausnehmungen zum Aufnehmen der Lanzette aufweist. Eine solche Struktur kann zum Beispiel durch Prägen oder durch Aufkleben einer Folie mit entsprechenden Ausnehmungen erzeugt werden.

Die auf dem ersten Teilkörper fixierte Lanzette, zumindest die Lanzettenspitze, ist durch eine Versiegelung geschützt, so lange sich das analytische Hilfsmittel in einem unbenutzten Zustand befindet. Durch die Versiegelung kann zumindest die Lanzettenspitze im unbenutzten Zustand bis unmittelbar vor der Benutzung steril (keimfrei) gehalten werden. Ferner erlaubt die Versiegelung keine Kontamination der Lanzettenspitze mit Bestandteilen der in dem Testfeld des Testelements enthaltenen Testchemie, da die Versiegelung die Lanzette vollständig von dem Testfeld trennt.

Das Testelement dient der Analyse der Blutprobe beziehungsweise der Probe aus interstitieller Flüssigkeit. Es enthält mindestens ein Testfeld mit einer Testchemie, die auf die zu untersuchende Probe abgestimmt ist. Das Testelement ist auf dem zweiten Teilkörper des erfindungsgemäßen analytischen Hilfsmittels angeordnet. Das Testelement beziehungsweise der zweite Teilkörper kann ferner ein Mittel zum Probentransport von einem Probenaufnahmeort, der zur Aufnahme der Probe von der mit der Lanzette perforierten Haut dient, zu dem Testfeld, auf dem die Analyse der Probe durchgeführt wird, tragen.

Die zwei Teilkörper sind über eine scharnierartige Verbindung miteinander verbunden. Die scharnierartige Verbindung ermöglicht ein Verschwenken der beiden Teilkörper gegeneinander. Im unbenutzten Zustand des erfindungsgemäßen analytischen Hilfsmittels liegen die beiden Teilkörper im Wesentlichen in einer gemeinsamen Ebene. In dieser im Folgenden als Anfangsposition bezeichneten Anordnung bilden die zwei Teilkörper einen im Wesentlichen ebenen Grundkörper und sind nicht gegeneinander verschwenkt (α = 0°). Die Lanzette liegt dann an dem ebenen Grundkörper an und ist durch die Versiegelung geschützt, so dass eine unerwünschte Verletzung durch die Lanzettenspitze vermieden wird. Bei der Verwendung des erfindungsgemäßen analytischen Hilfsmittels werden die beiden Teilkörper gegeneinander verschwenkt, wodurch u.a. die Lanzettenspitze zur Benutzung freigegeben werden kann. Beim Verschwenken der Teilkörper wird in diesem Fall die Versiegelung aufgetrennt. Das Auftrennen der Versiegelung erfolgt dabei zum Beispiel durch Zerreißen aufgrund einer beim Verschwenken der Teilkörper auf die Versiegelung wirkenden Zugkraft oder durch Zerschneiden der Versiegelung durch die Lanzettenspitze. Eine weitere Möglichkeit ist, dass die Versiegelung beim Verschwenken der Teilkörper an ihrer Verbindungsstelle mit den Teilkörpern (z.B. Klebeverbindung) abgetrennt wird und so die Lanzettenspitze freigegeben wird. Die Versiegelung kann aber auch beim Einsetzen des analytischen Hilfsmittels in ein Analysegerät durch eine dafür vorgesehene Einrichtung (z.B. Klinge) oder vor dem Einsetzen in das Analysegerät durch den Benutzer geöffnet werden.

Die scharnierartige Verbindung ist bei der vorliegenden Erfindung vorzugsweise so ausgebildet, dass die Teilkörper um einen Winkel α von mindestens 90° und bis zu 180° aus der Anfangsposition gegeneinander verschwenkt werden können. Bei einem Verschwenken um α = 180° aus der Anfangsposition sind die zwei Teilkörper zusammengeklappt, also nebeneinander und parallel zueinander angeordnet und an der scharnierartigen Verbindung miteinander verbunden.

Sobald die beiden Teilkörper gegeneinander verschwenkt sind und die Lanzettenspitze freigegeben ist, kann die Lanzettenspitze zur Perforation der Haut (zum Beispiel der Fingerbeere) eines Patienten dienen, um eine Probe aus Blut oder interstitieller Flüssigkeit zu gewinnen. Anschließend können die Teilkörper des analytischen Hilfsmittels wieder in die Anfangsposition zurückgeschwenkt werden, so dass die Lanzette erneut an dem Grundkörper anliegt und eine Verletzungsgefahr weitgehend ausgeschlossen ist. Zur Aufnahme der Probe kann dann der Probenaufnahmeort des analytischen Hilfsmittels an die Probe (zum Beispiel einen Blutstropfen) herangeführt werden, um diese auf das Testelement aufzunehmen, ggf. mit Hilfe des Mittels zum Probentransport zum Testfeld zu transportieren, wo sie mit der Testchemie reagiert, und sie dort zu analysieren.

Die vorliegende Erfindung weist u.a. folgende Vorteile auf:
- Das erfindungsgemäße analytische Hilfsmittel weist einen einfachen und kompakten Aufbau mit einer geringen Anzahl an Komponenten auf und kombiniert eine Vielzahl von Funktionen. Es ist für den Einsatz in kompakten automatisierten Analysegeräten geeignet.
- Die Lanzette ist durch die Versiegelung keimdicht verschlossen, so dass Keime bis unmittelbar vor der Benutzung der Lanzette nicht an die Lanzettenspitze gelangen können. Sie ist durch die Versiegelung auch vor Kontakt mit der Testchemie geschützt.
- Die zwei Teilkörper, von denen der eine die Lanzette und der andere das Testelement trägt, können getrennt voneinander vorkonfektioniert werden. Das Sterilisieren der Lanzette kann daher vor dem Verbinden der zwei Teilkörper mit der scharnierartigen Verbindung erfolgen (zum Beispiel durch γ-Bestrahlung), so dass die Testchemie nicht der gegebenenfalls schädlichen Sterilisierung ausgesetzt wird.
- Das erfindungsgemäße analytische Hilfsmittel kann eine ähnliche Form wie handelsübliche Teststreifen aufweisen, so dass es in einem an den Aufbau und die Funktionsweise des analytischen Hilfsmittels angepassten handelsüblichen Analysegerät eingesetzt werden kann.
- Der Benutzer des erfindungsgemäßen analytischen Hilfsmittels ist vor einer unbeabsichtigten Verletzung an der Lanzettenspitze weitgehend geschützt. Das analytische Hilfsmittel ermöglicht eine hohe Einstichgeschwindigkeit der Lanzettenspitze und eine kurze Verweilzeit der Lanzettenspitze in der Haut, so dass das Einstechen schmerzarm ist.

Gemäß einer bevorzugten Ausführungsform der vorliegenden Erfindung ist die scharnierartige Verbindung ein flexibler Streifen, der die zwei Teilkörper miteinander verbindet. Der flexible Streifen kann gegen die in einer Ebene mit ihren Kanten aneinander liegenden Teilkörper geklebt oder geschweißt werden. Er kann zum Beispiel aus einem Klebeband bestehen. Die scharnierartige Verbindung kann auch allein durch die Versiegelung gebildet werden. Eine weitere Möglichkeit ist die Ausführung der scharnierartigen Verbindung als Filmscharnier. Filmscharniere oder Filmgelenke sind Bandscharniere und haben keine mechanischen Teile. Ein Filmscharnier ist eine flexible, dünnwandige Gelenkrille zwischen zwei zu verbindenden Teilen.

Der Probenaufnahmeort ist vorzugsweise im Bereich eines der scharnierartigen Verbindung entgegengesetzten Endes des zweiten Teilkörpers angeordnet. Dadurch befindet sich der Probenaufnahmeort an einem exponierten und damit gut erreichbaren Ende des Grundkörpers, wenn die Teilkörper nicht gegeneinander verschwenkt sind und somit in einer Ebene liegen.

Die Lanzettenspitze ragt vorzugsweise im Bereich der scharnierartigen Verbindung über den ersten Teilkörper hinaus. Dadurch liegt sie an dem Grundkörper beziehungsweise an dem zweiten Teilkörper an, wenn sich das erfindungsgemäße analytische Hilfsmittel in der Anfangsposition befindet und ragt gut zugänglich und stechbereit über die Kante des ersten Teilkörpers hinaus, sobald die Teilkörper um die scharnierartige Verbindung um einen Winkel α ≥ 90° gegeneinander aus der Anfangsposition verschwenkt sind. Vorzugsweise ist die Lanzettenspitze daher im unbenutzten Zustand des analytischen Hilfsmittels im Wesentlichen parallel zu dem zweiten Teilkörper ausgerichtet.

Gemäß einer bevorzugten Ausfiihrungsform der vorliegenden Erfindung ist die Versiegelung ein taschenartiger Schutz, der die Lanzette im unbenutzten Zustand des analytischen Hilfsmittels teilweise oder vollständig umschließt. Im Falle eines nur teilweisen Umschließens ist zumindest die Lanzettenspitze durch den taschenartigen Schutz steril verschlossen. Der taschenartige Schutz wird zum Beispiel durch eine dünne Folie gebildet, die die Lanzette und insbesondere die Lanzettenspitze einhüllt, wobei die Folie längs der Lanzette zu einer Art Tasche verbunden ist. Die Folie wird dabei zum Beispiel vor der Montage der Lanzette auf dem ersten Teilkörper teilweise aufgesiegelt und nach der Montage der Lanzette über die Lanzettenspitze gefaltet und zum Beispiel mit Klebstoff am aufgesiegelten Teil der Folie so befestigt, dass sich die Lanzettenspitze in einem aus der Folie gebildeten taschenartigen Schutz befindet. Bevorzugt wird die Lanzette bei der Montage um ihre Längsachse so gedreht, dass eine Kante des Lanzettenschliffs möglichst senkrecht zur scharnierartigen Verbindung orientiert ist und von ihr weg weist.

Vorzugsweise ist die Versiegelung bei der Lanzettenspitze fest mit dem zweiten Teilkörper verbunden. Im Falle eines taschenartigen Schutzes kann dieser zum Beispiel an seiner Vorderkante bei der Lanzettenspitze mit dem zweiten Teilkörper verbunden sein. Somit überspannt die Versiegelung, die sowohl an dem ersten Teilkörper befestigt ist, als auch im Bereich der Lanzettenspitze mit dem zweiten Teilkörper verbunden ist, die scharnierartige Verbindung zwischen den beiden Teilkörpern. Sie ist vorzugsweise in der Anfangsposition vollständig ausgestreckt und wird beim Verschwenken der Teilkörper aus der Anfangsposition über die Kanten der Teilkörper bei der scharnierartigen Verbindung gespannt, so dass sie im Bereich der Lanzettenspitze zerreißt und diese zur Benutzung freigibt. Dazu ist die Versiegelung zumindest im Bereich der Lanzettenspitze aus einem spröden Material gefertigt. Spröde bedeutet in diesem Zusammenhang, dass das Material eine kleine Bruchdehnung und geringe Weiterreißfestigkeit aufweist. Ein bevorzugtes Material ist zum Beispiel geschäumtes orientiertes Polypropylen (OPP) der Fa. Huhtamaki Deutschland GmbH&Co. KG in Ronsberg (Allgäu). Durch die Materialwahl und die Orientierung des Lanzettenschliffs wird ein glattes Durchtrennen der Versiegelung erreicht. Weiterhin wird in einer bevorzugten Ausgestaltung der Erfindung die scharnierartige Verbindung durch die Versiegelung gebildet.

Das Testelement umfasst bei der vorliegenden Erfindung vorzugsweise Mittel zur elektrochemischen oder optischen Analyse einer auf dem Testfeld vorhandenen Probe. Die sensorische Erfassung eines Analyten auf einem Testelement mittels photometrischer oder elektrochemischer Verfahren erfolgt gemäß im Stand der Technik bekannter Verfahren. Im Falle, dass ein elektrochemischer Nachweis eines Analyten in der Probe erfolgen soll, ist der zweite Teilkörper mit elektrischen Verbindungen versehen. Diese elektrischen Verbindungen können beispielsweise durch Aufsputtern einer Goldschicht und das Formen von Elektroden, Leiterbahnen und Kontaktpads aus der Goldschicht mittels Laserablation hergestellt werden. Die Elektroden können mit Reagenz- und Abdeckschichten überzogen werden. Im Falle, dass ein optischer Nachweis eines Analyten in der Probe erfolgen soll, ist der zweite Teilkörper mit den notwendigen optischen Elementen ausgestattet, zum Beispiel einem lichtdurchlässigen Fenster.

Gemäß einer Ausführungsform der vorliegenden Erfindung weist das erfindungsgemäße analytische Hilfsmittel eine Ausnehmung in dem ersten Teilkörper zum Eingreifen eines Führungselements in einem Analysegerät auf, durch das das analytische Hilfsmittel beim Einstich der Lanzette und bei der Probenaufnahme an eine Probenentnahmestelle herangeführt wird. Mit Hilfe des in die Ausnehmung eingreifenden Führungselements kann das erfindungsgemäße analytische Hilfsmittel in dem Analysegerät ohne einen manuellen Eingriff des Benutzers in die entsprechende Position geführt werden.

Vorzugsweise enthält das erfindungsgemäße analytische Hilfsmittel ferner eine Abdeckung, die auf dem analytischen Hilfsmittel angeordnet ist und die mit dem ersten Teilkörper fest und mit dem zweiten Teilkörper über eine Haftzone lösbar und wiederverbindbar verbunden ist. Die Abdeckung ist vorzugsweise nur an dem der scharnierartigen Verbindung entgegengesetzten Ende des ersten Teilkörpers fest verbunden und liegt auf den beiden Teilkörpern auf, so lange das analytische Hilfsmittel nicht benutzt wird. Die Abdeckung kann während der Benutzung zumindest von dem zweiten Teilkörper und der Lanzettenspitze weggeklappt werden, so dass sie einer Benutzung des analytischen Hilfsmittels nicht im Wege steht und nach der Benutzung wieder den gesamten Grundkörper abdecken, um die Lanzette unzugänglich aufzubewahren. Als Material für die Abdeckung wird vorzugsweise Polyester oder Polycarbonat gewählt. Die Abdeckung hat bevorzugt eine Dicke von 100 bis 150 µm. Sie kann dieselbe Länge wie der Grundkörper besitzen oder länger als der Grundkörper ausgeführt sein, so dass sie über das der scharnierartigen Verbindung entgegengesetzte Ende des zweiten Teilkörpers in der Anfangsposition hinausragt. An dem zweiten Teilkörper ist die Abdeckung in der Anfangsposition lösbar mit Hilfe der Haftzone verbunden. Die Haftzone kann zum Beispiel eine leicht lösbare und wiederverbindbare Klebeschicht aufweisen, an der die Abdeckung beziehungsweise der zweite Teilkörper haftet. Die Haftzone kann auf dem zweiten Teilkörper oder auf der Abdeckung angeordnet sein. Durch die Abdeckung kann in vorteilhafter Weise eine unbeabsichtigte Verletzung des Benutzers oder Kontamination durch Probenreste vermieden werden.

Gemäß einer bevorzugten Ausführungsform der vorliegenden Erfindung ist das Mittel zum Probentransport eine Kapillare. Eine Kapillare in Form eines Kapillarspalts kann zum Beispiel durch ein strukturiertes Spacer-Klebeband und eine hydrophile Deckelfolie gebildet werden, die auf dem zweiten Teilkörper befestigt sind. Das Mittel zum Probentransport kann aber auch jedes andere, dem Fachmann bekannte, zum Probentransport geeignete Mittel sein, beispielsweise kapillaraktives Material oder ein Docht. Die Probe kann sich bei dem erfindungsgemäßen analytischen Hilfsmittel jedoch auch ohne ein zusätzlich vorgesehenes Mittel zum Probentransport von dem Probenaufnahmeort auf dem Testfeld verteilen, z.B. falls das Testfeld an den Probenaufnahmeort grenzt und ein saugfähiges Material enthält.

Die Erfindung bezieht sich weiterhin auf ein Verfahren zur Herstellung von erfindungsgemäßen analytischen Hilfsmitteln, wobei die ersten Teilkörper aus Abschnitten eines ersten biegsamen Bandes und die zweiten Teilkörper aus Abschnitten eines zweiten biegsamen Bandes gefertigt werden, und die beiden biegsamen Bänder mit Hilfe von Pilotlöchern relativ zueinander ausgerichtet werden, bevor sie miteinander zu Grundkörpern verbunden und die Abschnitte vereinzelt werden. Die biegsamen Bänder müssen einerseits so starr sein, dass ein Verschwenken der daraus gefertigten Teilkörper zum Freisetzen der Lanzettenspitze möglich ist und müssen andererseits so flexibel sein, dass eine Rollenproduktion möglich ist. Geeignete biegsame Bänder sind beispielsweise Polyesterfolien, z. B. die Polyesterfolie "Melinex", Hersteller DuPont Teijin Films, mit einer Dicke von ca. 350 µm, oder ähnliche Polymerfolien. Die zwei biegsamen Bänder können aus einem biegsamen Band durch Zerschneiden hergestellt werden. Die Lanzetten und Versiegelungen werden an dem ersten biegsamen Band befestigt. Das erste biegsame Band wird dann aufgerollt und einer Sterilisierung, insbesondere einer Strahlensterilisation unterworfen. Eine Strahlensterilisation kann zum Beispiel mit einer Dosis von 25 kGray durchgeführt werden. Nachdem die Lanzetten und die Versiegelungen jeweils auf einem Abschnitt des ersten biegsamen Bandes befestigt und sterilisiert wurden und die Testelemente jeweils auf einem Abschnitt des zweiten biegsamen Bandes befestigt wurden, können die beiden biegsamen Bänder miteinander verbunden werden. Dazu müssen sie passgenau miteinander verbunden werden. Die Bänder werden daher unter einer regelbaren Spannung gehalten, die so geregelt wird, dass die in beiden Bändern vorhandenen Pilotlöcher sich immer innerhalb einer zulässigen Toleranz gegenüberliegen, bevor die Bänder miteinander über eine scharnierartige Verbindung zum Beispiel durch ein Klebeband zu einem einzigen biegsamen Band verbunden werden. Dann wird die Versiegelung mindestens mit einer Kante an dem zweiten Teilkörper durch Kleben oder Schweißen befestigt. Anschließend werden die einzelnen erfindungsgemäßen analytischen Hilfsmittel von dem Band abgeschnitten. Der Schnitt zum Vereinzeln der analytischen Hilfsmittel wird mittig zwischen je zwei Lanzetten parallel zu diesen geführt. Vor oder nach dem Vereinzeln der analytischen Hilfsmittel können die Bereiche, die die Pilotlöcher aufweisen, zum Beispiel durch Abschneiden entfernt werden.

Eine andere Möglichkeit der Herstellung der erfindungsgemäßen analytischen Hilfsmittel aus zwei biegsamen Bändern ist, dass die Abschnitte des ersten biegsamen Bandes mit den versiegelten und sterilisierten Lanzetten und die Abschnitte des zweiten biegsamen Bandes mit den Testelementen zuerst getrennt vereinzelt werden und anschließend jeweils ein Abschnitt der zwei Bänder über scharnierartige Verbindungen zu jeweils einem Grundkörper miteinander verbunden werden (gegebenenfalls ebenfalls unter Verwendung jeweils mindestens eines Pilotlochs).

Gemäß einer bevorzugten Ausführung der vorliegenden Erfindung wird die Versiegelung jedes einzelnen analytischen Hilfsmittels nach dem Vereinzeln in einem Vakuum auf Dichtigkeit kontrolliert, so dass eine Beschädigung der Versiegelung bei dem Schnitt zum Vereinzeln der analytischen Hilfsmittel erkannt wird. Dazu können die einzelnen analytischen Hilfsmittel in eine Kammer eingebracht werden, die evakuiert wird. Die durch die Versiegelung eingeschlossene Luft bläht die Versiegelung mit abnehmendem Außendruck zunehmend auf. Bei einer beschädigten Versiegelung bläht sich diese nicht oder nur unvollständig auf. Dies kann durch ein automatisches Bildverarbeitungssystem erfasst und ausgewertet werden. Defekte analytische Hilfsmittel werden ausgesondert. Sie können mit Hilfe der Bildauswertung auf die Fehlerursache hin untersucht werden. Dies erlaubt eine schnelle Reaktion auf und Beseitigung der Fehlerursachen.

Die fertigen erfindungsgemäßen analytischen Hilfsmittel können erfindungsgemäß einzeln oder zu mehreren in geeigneten Behältern oder Magazinen verpackt aufbewahrt werden. Als Magazin kann beispielsweise ein System verwendet werden, wie es in der US 6,497,845 B1 beschrieben wird. Eine Anpassung eines solchen Magazins an die spezifischen Erfordernisse des erfindungsgemäßen analytischen Hilfsmittels kann der Fachmann durchführen.

Die Erfindung bezieht sich weiterhin auf ein Verfahren zur Analyse einer Probe mit einem erfindungsgemäßen analytischen Hilfsmittel mit den Schritten
A) Einsetzen eines analytischen Hilfsmittels in ein Analysegerät,
   A1) alternativ oder zusätzlich: Einsetzen eines Magazins in ein Analysegerät; Bereitstellen eines analytischen Hilfsmittels durch das Analysegerät,
B) Verschwenken der zwei Teilkörper gegeneinander aus einer Anfangsposition um einen Winkel α, wobei die Versiegelung aufgetrennt und die Lanzettenspitze über den ersten Teilkörper hinausragend zur Benutzung freigegeben wird,
C) Einstich der Lanzettenspitze in eine Probenentnahmestelle,
D) Schwenken der zwei Teilkörper in die Anfangsposition oder in eine andere Probenentnahmeposition,
E) Aufnahme der Probe an der Probenentnahmestelle auf den Probenaufnahmeort des analytischen Hilfsmittels, so dass die Probe auf das Testfeld gelangt und
F) Analyse der Probe auf dem Testfeld.
G) optional zusätzlich: Auswurf eines gebrauchten analytischen Hilfsmittels
   G1) (alternativ zu G) Rückführung des gebrauchten analytischen Hilfsmittels in ein Magazin.

Vorzugsweise tritt die Lanzettenspitze in Schritt C) aus einer Öffnung des Analysegeräts aus und der Probenaufnahmeort tritt in Schritt E) aus derselben Öffnung aus.

Anhand der Zeichnung wird die Erfindung nachstehend näher erläutert.

Es zeigen
- Figur 1: eine schematische Darstellung eines erfindungsgemäßen analytischen Hilfsmittels,
- Figur 2: schematisch die mögliche Funktionsweise eines erfindungsgemäßen analytischen Hilfsmittels,
- Figur 3: schematisch eine andere mögliche Funktionsweise eines erfindungsgemäßen analytischen Hilfsmittels und
- Figuren 4 bis 11: einen Bewegungsablauf eines erfindungsgemäßen analytischen Hilfsmittels in einem Analysegerät.

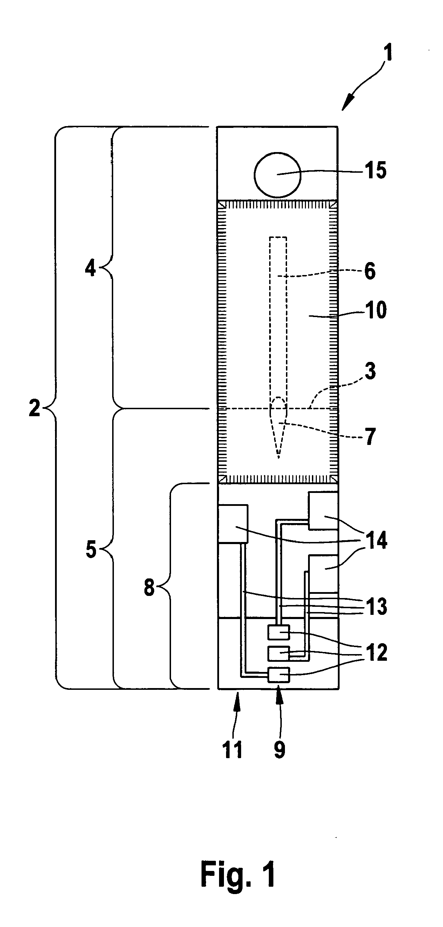

Figur 1 zeigt eine schematische Darstellung eines erfindungsgemäßen analytischen Hilfsmittels.

Das analytische Hilfsmittel 1 umfasst einen Grundkörper 2 mit zwei über eine scharnierartige Verbindung 3 verbundenen Teilkörpern 4, 5. Der erste Teilkörper 4 trägt eine Lanzette 6, die eine Lanzettenspitze 7 aufweist. Der zweite Teilkörper 5 trägt ein Testelement 8, das ein (nicht dargestelltes) Testfeld enthält. Ein (nicht dargestelltes) Mittel zum Probentransport in Form einer Kapillare dient zum Transport einer Probenflüssigkeit von dem Probenaufnahmeort 9 zu dem Testfeld des Testelements 8. Der Probenaufnahmeort 9 ist im Bereich eines der scharnierartigen Verbindung 3 entgegengesetzten Endes 11 des zweiten Teilkörpers 5 angeordnet.

Figur 1 zeigt das analytische Hilfsmittel im unbenutzten Zustand. Die zwei Teilkörper 4, 5 sind daher in einer gemeinsamen Ebene angeordnet. Die Lanzette 6 ist durch eine Versiegelung 10 geschützt. Die Versiegelung 10 ist mit dem ersten Teilkörper 4 fest verbunden. Sie umschließt die gesamte Lanzette 6 und insbesondere die Lanzettenspitze 7, die im Bereich der scharnierartigen Verbindung 3 über den ersten Teilkörper 4 hinausragt und im Wesentlichen parallel zu dem zweiten Teilkörper 5 ausgerichtet ist. Im Bereich der Lanzettenspitze 7 ist die Versiegelung 10, die die scharnierartige Verbindung 3 überspannt, fest mit dem zweiten Teilkörper 5 verbunden.

Die zwei Teilkörper 4, 5 sind bei der Verwendung des erfindungsgemäßen analytischen Hilfsmittels 1 um die scharnierartige Verbindung 3 aus der Ebene gegeneinander verschwenkbar. Beim Verschwenken der zwei Teilkörper 4, 5 wird die Versiegelung 10 im Bereich der Lanzettenspitze 7 aufgetrennt, so dass die Lanzettenspitze 7 zur Benutzung freigegeben wird.

Das in Figur 1 dargestellte Testelement 8 umfasst Mittel zur elektrochemischen Analyse in Form von Elektroden 12, elektrischen Leiterbahnen 13 und Kontaktpads 14. Des Weiteren enthält das analytische Hilfsmittel 1 eine Ausnehmung 15 in dem ersten Teilkörper 4 zum Eingreifen eines (nicht dargestellten) Führungselements in einem Analysegerät, durch das das analytische Hilfsmittel 1 beim Einstich der Lanzette 6 und bei der Probenaufnahme an eine Probenentnahmestelle (zum Beispiel an einer Fingerbeere eines Patienten) herangeführt wird.

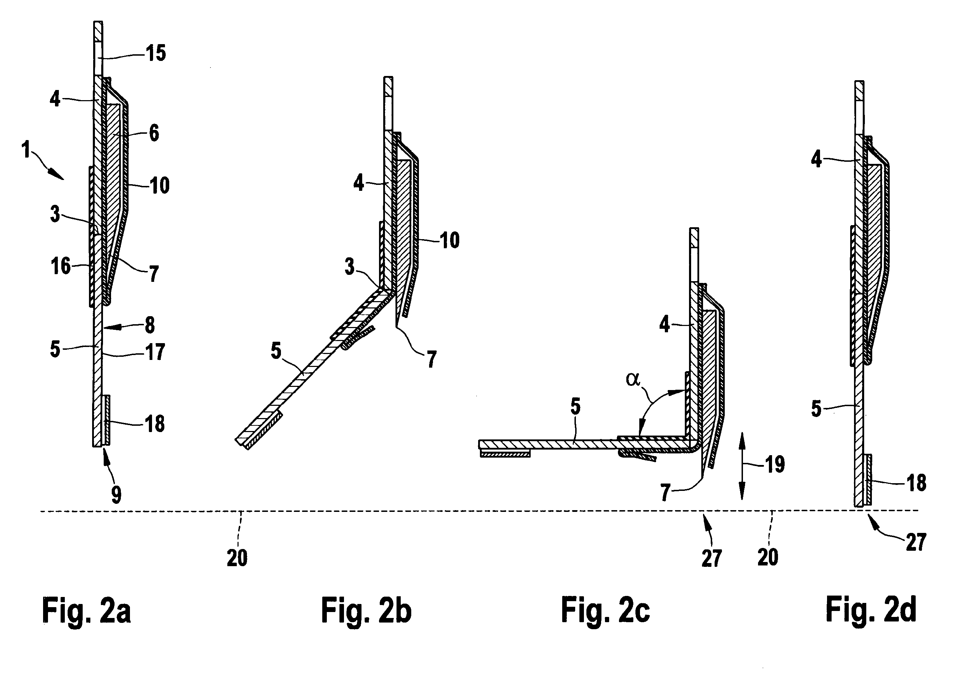

Figur 2 zeigt schematisch die Funktionsweise eines erfindungsgemäßen analytischen Hilfsmittels, insbesondere beim Einstich der Lanzette und bei der Probenaufnahme.

Ein erfindungsgemäßes analytisches Hilfsmittel 1 ist in Figur 2 seitlich im Schnitt dargestellt. Es umfasst zwei Teilkörper 4, 5, die über eine scharnierartige Verbindung 3 zu einem Grundkörper 2 verbunden sind, wobei der erste Teilkörper 4 eine Ausnehmung 15 zur Führung des analytischen Hilfsmittels 1 aufweist. Eine am ersten Teilkörper 4 befestigte Lanzette 6 ist im unbenutzten Zustand (Figur 2 (a)) durch eine taschenartige Versiegelung 10 geschützt. Ein flexibler Streifen 16, der die zwei Teilkörper 4, 5 miteinander verbindet, dient als scharnierartige Verbindung 3. Ein auf dem zweiten Teilkörper 5 angeordnetes Testelement 8 umfasst ein Testfeld 17, das über einen als Mittel zum Probentransport 18 dienenden Kapillarspalt mit dem Probenaufnahmeort 9 verbunden ist. Die gestrichelte Linie stellt das Hautniveau 20 dar.

Nachfolgend wird anhand von Figur 2 der mögliche Ablauf einer Messung, zum Beispiel von Blutglukose beschrieben. In Figur 2 (a) ist ein erfindungsgemäßes analytisches Hilfsmittel im unbenutzten Zustand dargestellt. Die zwei Teilkörper 4, 5 befinden sich in einer Ebene und die Lanzette 6 ist durch die Versiegelung geschützt. Das streifenförmige analytische Hilfsmittel 1 wird als Einzelstreifen oder in einem Magazin gemeinsam mit einer Vielzahl von Streifen in ein geeignetes (nicht dargestelltes) Messgerät eingesetzt. Das Gerät wird zum Beispiel mit einem Konus gegen einen Finger eines Benutzers gedrückt, wodurch das Gerät eingeschaltet wird und der Ablauf ohne weitere Benutzerinteraktion erfolgen kann. Ein erfindungsgemäßes analytisches Hilfsmittel 1 wird aus einem Magazin freigesetzt und in Richtung Finger transportiert. Wird statt eines Magazins ein einzeln eingesetztes analytisches Hilfsmittel 1 verwendet, so ist der Bewegungsablauf vergleichbar.

In Figur 2 (b) ist das Verschwenken der beiden Teilkörper 4, 5 gegeneinander um die scharnierartige Verbindung dargestellt. An einer definierten Stelle (der scharnierartigen Verbindung 3) wird das streifenförmige analytische Hilfsmittel 1 abgeknickt. Das Abknicken erfolgt dadurch, dass das analytische Hilfsmittel 1 entweder beim Transport in dem Gerät, durch eine Klappbewegung einer Geräteverschlusskappe oder durch eine sonstige, auf die Knickstelle einwirkende Kraft abgeknickt wird. Dabei schneidet die Lanzettenspitze 7 durch die als Versiegelung 10 dienende Sterilschutzhülle.

Figur 2 (c) zeigt das analytische Hilfsmittel 1 beim Einstich der Lanzettenspitze 7, wobei die Teilkörper 4, 5 um α = 90° gegeneinander verschwenkt sind. Sobald der Streifen um mindestens 90° abgeknickt ist, ragt die Lanzettenspitze 7 über die Streifenkontur hinaus. Durch eine schnelle Vorwärts/Rückwärtsbewegung des Streifens in Pfeilrichtung 19 wird die Lanzettenspitze 7 aus einer (nicht dargestellten) Öffnung des Gerätes in die Haut des aufgelegten Fingers gestochen. Diese schnelle Bewegung kann durch eine Federmechanik, durch einen Magnetaktuator oder durch einen Motorantrieb, gegebenenfalls in Verbindung mit einer Schwungmasse, erfolgen. Der Streifen wird nach dem Stechen so weit in das Gerät zurückgezogen, so dass er wieder im ungeknickten Zustand vorliegt.

In Figur 2 (d) ist die Probenaufnahme gezeigt. Dazu wird der Weg im Gerät zum Beispiel über eine Weiche geändert, so dass der Streifen beim erneuten Herausschieben ungeknickt mit der Öffnung der Kapillare 18 aus dem Gerät austritt. Damit trifft der saugfähige Probenaufnahmeort genau auf einen inzwischen aus der Haut ausgetretenen Blutstropfen. Am Ende der Austrittsbewegung werden die Kontaktpads auf dem Streifen von der Messelektronik kontaktiert (nicht dargestellt). Dies erfolgt durch Schleifkontakte oder Aufsetzkontakte. Bei optischer Auswertung startet zum Beispiel die Reflexionsmessung. Sobald das Probenvolumen aufgesaugt ist, kann der Streifen wieder in das Gerät zurückgezogen werden. Damit wird eine Störung der Messung durch Bewegungen unterbunden. Der Nutzer kann ein akustisches Signal erhalten, dass er das Gerät vom Finger nehmen kann. Gleichzeitig erfolgt die Messung einer optischen Veränderung, eines elektrischen Stromes oder eines elektrischen Potentials vergleichbar mit der Messung bei konventionellen Teststreifen. Nach dem Ende der Messung kann das gebrauchte analytische Hilfsmittel 1 ausgeworfen oder in ein Magazin zurückgezogen werden. Der Probenaufnahmeort kann bei dem erfindungsgemäßen analytischen Hilfsmittel z.B. an dem der scharnierartigen Verbindung abgewandten Ende des zweiten Teilkörpers oder an einer Längsseite des zweiten Teilkörpers angeordnet sein.

Das erfindungsgemäße Verfahren läuft demnach wie folgt ab:
A) Einsetzen eines analytischen Hilfsmittels 1 in ein Analysegerät,
B) Verschwenken der zwei Teilkörper 4, 5 gegeneinander aus einer Anfangsposition um einen Winkel α, wobei die Versiegelung 10 aufgetrennt und die Lanzettenspitze über den ersten Teilkörper 4 hinausragend zur Benutzung freigegeben wird,
C) Einstich der Lanzettenspitze 7 in eine Probenentnahmestelle 27 (siehe z. B. unten, Fig.3),
D) Aufnahme der Probe an der Probenentnahmestelle 27 auf den Probenaufnahmeort 9 des analytischen Hilfsmittels 1, so dass die Probe auf das Testfeld 17 gelangt und
E) Analyse der Probe auf dem Testfeld 17.

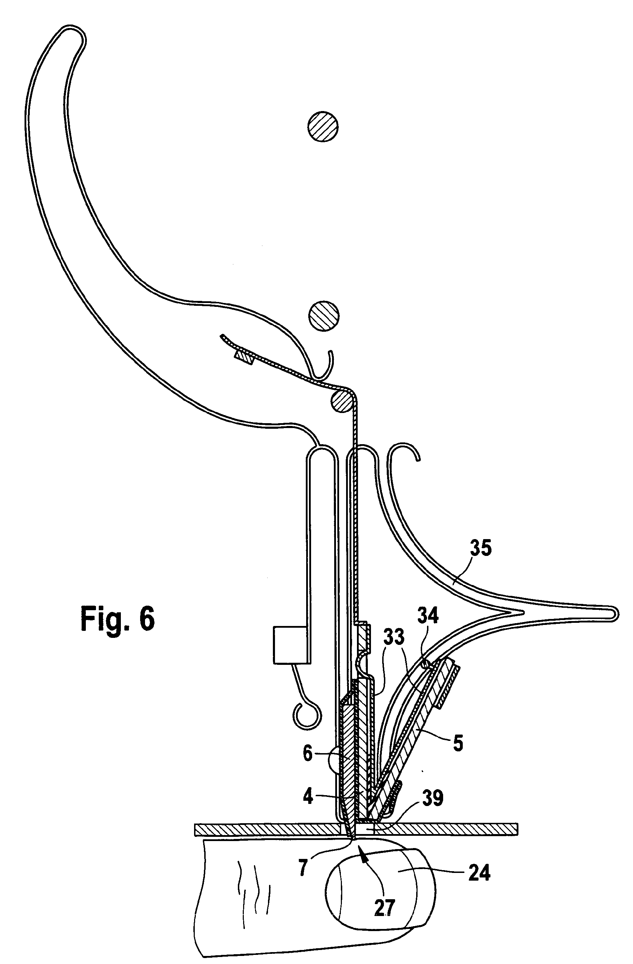

In Schritt C) tritt die Lanzettenspitze 7 aus einer Öffnung 39 (siehe z. B. unten, Fig. 6) des Analysegeräts aus und in Schritt E) tritt der Probenaufnahmeort 9 aus derselben Öffnung 39 aus. In Schritt D) des erfindungsgemäßen Verfahrens werden die zwei Teilkörper 4, 5 vorzugsweise so geschwenkt, dass sie sich (wie im unbenutzten Zustand) in einer gemeinsamen Ebene befinden (ungeknickter Streifen). In dieser Probenaufnahmeposition, die der Anfangsposition entsprechen kann, wird die Probe am Probenaufnahmeort auf das erfindungsgemäße analytische Hilfsmittel 1 aufgenommen.

Figur 3 zeigt schematisch eine andere Funktionsweise eines erfindungsgemäßen analytischen Hilfsmittels, insbesondere beim Einstich der Lanzettenspitze und bei der Probenaufnahme.

Das in Figur 3 gezeigte erfindungsgemäße analytische Hilfsmittel 1 entspricht in seinem Aufbau dem in Figur 2 dargestellten analytischen Hilfsmittel.

In Figur 3 (a) befindet sich das analytische Hilfsmittel 1 in einer Anfangsposition in einem Analysegerät, in der die zwei Teilkörper 4, 5 in einer Ebene angeordnet sind. Die Versiegelung 10 ist intakt. Ein zu dem Analysegerät gehörender Schieber 21 ist oberhalb der scharnierartigen Verbindung 3 angeordnet.

Figur 3 (b) zeigt das Verschwenken der zwei Teilkörper 4, 5 gegeneinander aus der Anfangsposition. Der Schieber 21 übt dazu eine Kraft von oben auf den Grundkörper 2 des analytischen Hilfsmittels 1 im Bereich der scharnierartigen Verbindung 3 aus. Gleichzeitig liegen die beiden Teilkörper 4, 5 in einem bestimmten Abstand zu der scharnierartigen Verbindung 3 auf jeweils einem Umlenker 22, 23 auf Dadurch knickt das streifenförmige analytische Hilfsmittel 1 an der scharnierartigen Verbindung. Die Versiegelung 10 wird aufgetrennt und die Lanzettenspitze 7 wird über den ersten Teilkörper 4 hinausragend freigegeben.

In Figur 3 (c) ist der Einstich der Lanzettenspitze 7 in einen Finger 24 eines Patienten dargestellt. Dabei sind die zwei Teilkörper 4, 5 um fast α = 180° gegenüber der Anfangsposition verschwenkt. Der Schieber 21 weist einen Vorsprung 25 auf, der in dieser Position in die Ausnehmung 15 in dem ersten Teilkörper 4 eingreift. Der Schieber 21 dient beim Einstich der Lanzettenspitze 7 in den Finger 24 und bei den nachfolgenden Schritten als Führungselement 26, durch das das analytische Hilfsmittel 1 beim Einstich der Lanzette 6 und bei der Probenaufnahme an die Probenentnahmestelle 27 herangeführt wird. Der Einstich in die Probenentnahmestelle 27 auf dem Finger 24 erfolgt in Bewegungsrichtung 19.

Figur 3 (d) zeigt das Zurückziehen des analytischen Hilfsmittels 1 nach dem Einstich. Es wird zurückgezogen, bis ein weiterer Umlenker 28 zwischen die zwei zusammengeklappten Teilkörper 4, 5 geschoben wird, und diese wieder auseinanderschwenkt. Dabei bleibt der erste Teilkörper 4 weiterhin über den Eingriff des Vorsprungs 25 in die Ausnehmung 15 mit dem Schieber 21 verbunden.

In Figur 3 (e) sind die Teilkörper 4, 5 in eine Probenaufnahmeposition geschwenkt, in der sich die Teilkörper 4, 5 in einer Ebene und das analytische Hilfsmittel 1 senkrecht zur in Figur 3 (a) gezeigten Anfangsposition befinden.

Figur 3 (f) zeigt die Probenaufnahme. Der Probenaufnahmeort 9 auf dem Testelement 8 wird an die Probenentnahmestelle 27 auf dem Finger 24 herangefahren, an der sich ein Blutstropfen 29 gebildet hat. Das Blut wird am Probenaufnahmeort 9 aufgenommen und mit Hilfe der Kapillare 18 zum Testfeld 17 transportiert, wo die Probe, wie oben zu Figur 2 beschrieben, analysiert wird.

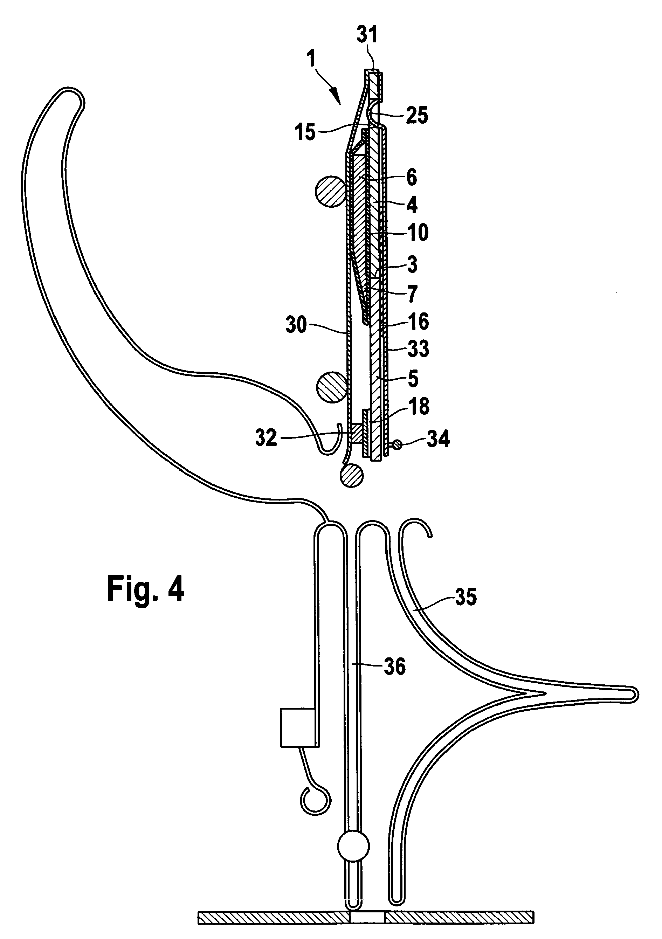

In den Figuren 4 bis 11 ist schematisch ein Bewegungsablauf eines erfindungsgemäßen analytischen Hilfsmittels in einem Analysegerät dargestellt.

Das erfindungsgemäße analytische Hilfsmittel ist im Schnitt dargestellt. Der Aufbau des analytischen Hilfsmittels 1 entspricht weitgehend dem Aufbau der bezüglich Figuren 2 und 3 beschriebenen analytischen Hilfsmittel 1, mit dem Unterschied, dass es zusätzlich eine Abdeckung 30 aufweist, die auf dem analytischen Hilfsmittel 1 angeordnet ist. Die Abdeckung 30 ist an ihrem einen Ende 31 mit dem ersten Teilkörper 4 fest verbunden. Mit dem zweiten Teilkörper 5 ist die Abdeckung 30 über eine Haftzone 32 lösbar und wieder verbindbar verbunden. Die Abdeckung 30 schützt vor unbeabsichtigten Verletzungen durch die Lanzette 6, insbesondere nach der Benutzung des analytischen Hilfsmittels 1.

In Figur 4 ist das analytische Hilfsmittel 1 in seiner Anfangsposition in einem schematisch dargestellten Analysegerät gezeigt. Das streifenförmige analytische Hilfsmittel 1 wird zum Beispiel durch einen Schlitz manuell in das Gerät eingeführt. Die Öffnung des Schlitzes ist deutlich größer als der Streifenquerschnitt und verengt sich auf einen Querschnitt, den das analytische Hilfsmittel 1 noch ohne wesentliche Reibung passieren kann. Dadurch wird die leichte Handhabung sichergestellt und der Anwender hat keine Schwierigkeiten, die Öffnung zu treffen. Es ist allerdings notwendig, den Streifen in der richtigen Orientierung einzuführen. Dazu können auf der Oberseite des analytischen Hilfsmittels 1 große, eindeutige Markierungen aufgedruckt werden. Im Analysegerät können geeignete optische oder mechanische oder andere Sensoren angebracht sein, die bei fehlerhafter Orientierung ein Warnsignal auslösen oder die Einführung des Streifens blockieren. Im Schlitz wird der Streifen über eine kurze Strecke (zum Beispiel von 5 mm) geradeaus geführt. Dies soll sicherstellen, dass das analytische Hilfsmittel 1 für die nachfolgenden Schritte richtig ausgerichtet ist, gleichgültig, unter welchem Winkel es in den Schlitz eingeführt wurde. In der Anfangsposition gemäß Figur 4 ist das analytische Hilfsmittel 1 in eine Halterung 33 in dem Analysegerät eingesetzt. Die Halterung 33 umfasst einen Vorsprung 25, der in die Ausnehmung 15 eingreift. Des Weiteren weist die Halterung 33 einen Mitnehmer 34 auf, der mit Führungsschienen 35, 36 in dem Analysegerät zusammenwirken kann. Die Halterung 33 ist ferner in dem Bereich, in dem das eingesetzte analytische Hilfsmittel 1 die scharnierartige Verbindung 3 hat, ebenfalls knickbar. In der Anfangsposition des analytischen Hilfsmittels 1 in dem Analysegerät sind die zwei Teilkörper 4, 5 des analytischen Hilfsmittels 1 in einer gemeinsamen Ebene angeordnet und die Abdeckung 30 bedeckt das analytische Hilfsmittel 1 auf einer Seite vollständig.

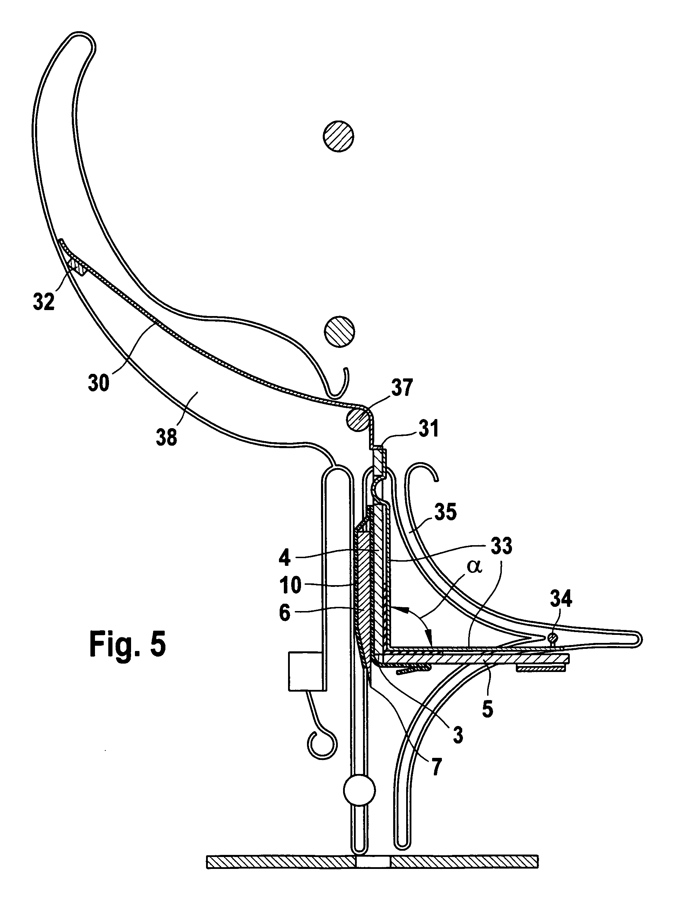

Figur 5 zeigt die Bewegung des analytischen Hilfsmittels 1 aus der Anfangsposition in Richtung zur Probenentnahmestelle. Der Mitnehmer 34 läuft dabei entlang der ersten Führungsschiene 35, die so ausgebildet ist, dass die in der Halterung 33 gehaltenen Teilkörper 4, 5 des analytischen Hilfsmittels 1 gegeneinander verschwenkt werden. In Figur 5 sind sie bereits um einen Winkel von α = 90° aus der Anfangsposition bewegt. Die Lanzette 6 hat mittels der Lanzettenspitze 7 die Versiegelung 10 aufgetrennt und die Lanzettenspitze 7 ragt über den ersten Teilkörper 4 hinaus. Während die Teilkörper 4, 5 verschwenkt und die Lanzettenspitze 7 freigegeben werden, wird die Abdeckung 30 über ein Leitelement 37 von dem Grundkörper 2 seitlich in einen separaten Kanal 38 weggeführt. Dazu wird die Abdeckung 30 mitsamt Haftzone 32 von dem analytischen Hilfsmittel 1 gelöst, so dass sie nur noch an dem Ende 31 mit dem ersten Teilkörper 4 verbunden ist.

Die Abdeckung 30 kann so geführt werden, dass sie eine Federwirkung entfaltet, die den Grundkörper 2 des analytischen Hilfsmittels 1 gegen einen in dem Analysegerät vorhandenen Anschlag drückt oder die das analytische Hilfsmittel 1 nach Beendigung des Messvorgangs auswirft.

Figur 6 zeigt den Einstich der Lanzettenspitze 7 in einen Finger 24. Dazu läuft der Mitnehmer 34 noch weiter in der ersten Führungsschiene 35 und nimmt die Halterung 33 mitsamt analytischem Hilfsmittel 1 mit, so dass die beiden Teilkörper 4, 5 bis zu einem Winkel von ca. 140° aus der Anfangsposition verschwenkt sind. Die Lanzettenspitze 7 tritt aus der Öffnung 39 aus dem Analysegerät aus und perforiert die Haut des Fingers 24.

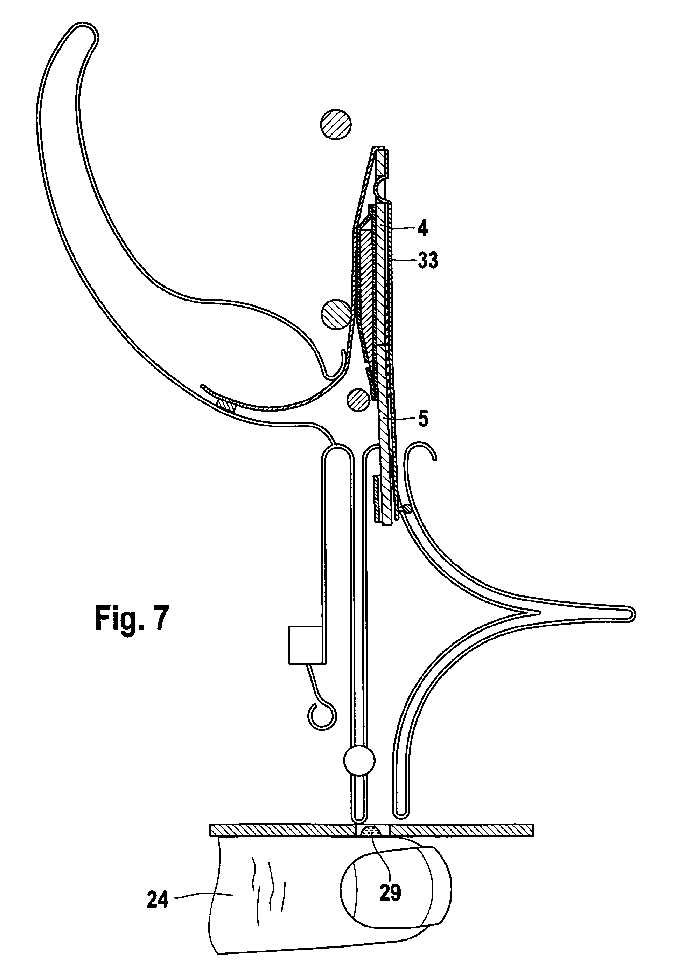

Gemäß Figur 7 wird das analytische Hilfsmittel 1 nach dem Einstich zurückgezogen, bis die beiden Teilkörper 4, 5 sich wieder in einer gemeinsamen Ebene befinden und die Halterung 33 ungeknickt vorliegt. Auf dem Finger 24 bildet sich während dessen ein Blutstropfen 29.

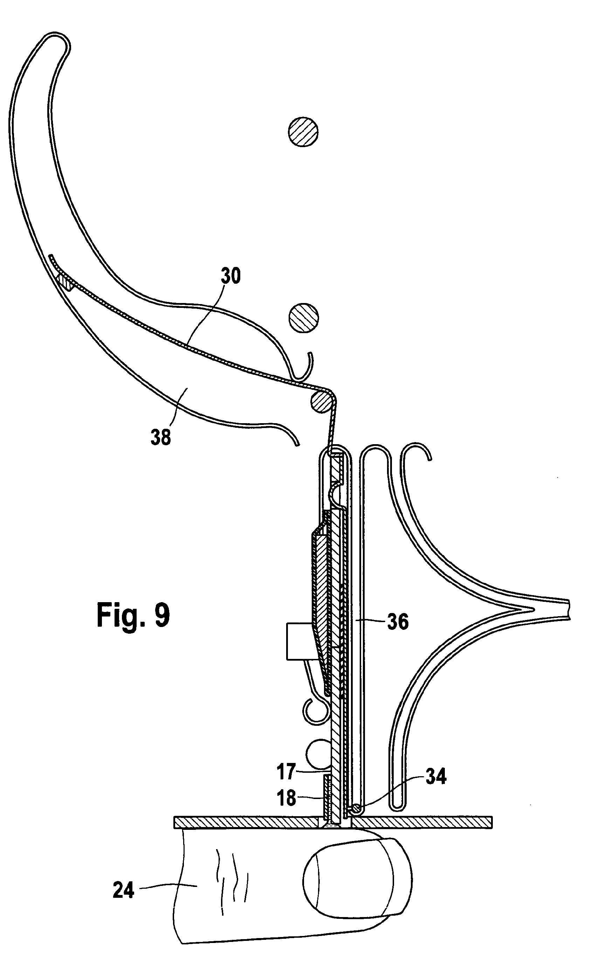

Figuren 8 und 9 zeigen, wie das erfindungsgemäße analytische Hilfsmittel 1 zur Probenaufnahme in dem Analysegerät bewegt wird. Der Mitnehmer 34 wird aus der ersten Führungsschiene 35 herausbewegt. Das analytische Hilfsmittel 1 wird in dem Analysegerät (zum Beispiel mittels einer Weiche) seitlich in eine neue in Figur 8 dargestellte Position verschoben. Dann wird der Probenaufnahmeort 9 zu dem Finger 24 hin bewegt, wobei der Mitnehmer 34 in der zweiten Führungsschiene 36 geführt wird. Bei der Probenaufnahme (Figur 9), die durch die Öffnung 39 in dem Analysegerät erfolgt, steigt die Blutprobe in dem kapillarförmigen Mittel zum Probentransport 18 auf, bis sie das Testfeld 17 erreicht. Während der Probenaufnahme ist die Abdeckung 30 von dem analytischen Hilfsmittel 1 weggeklappt und in dem Kanal 38 positioniert.

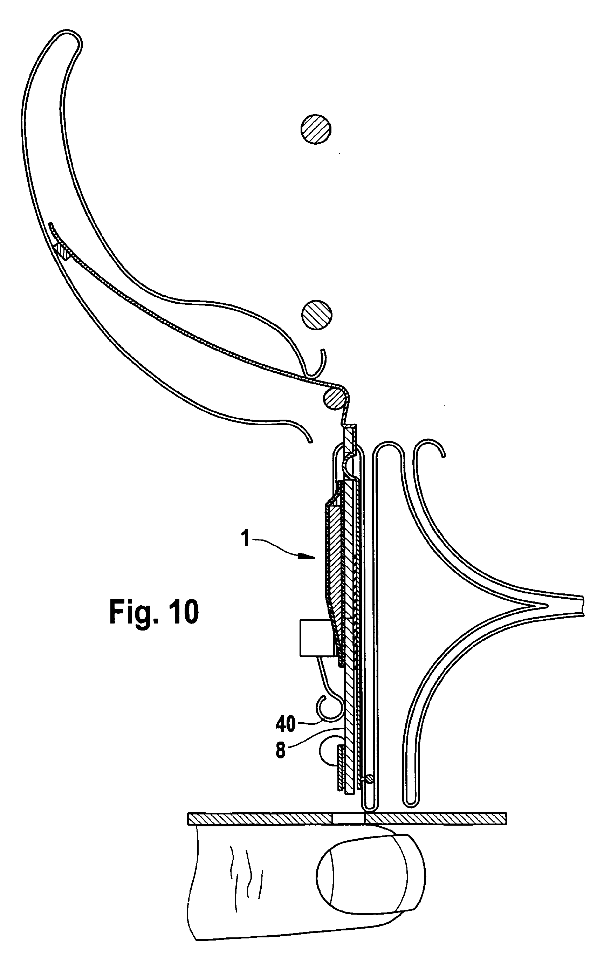

Figur 10 zeigt das erfindungsgemäße analytische Hilfsmittel 1 in einer Messposition. Zur Durchführung einer elektrochemischen Analyse wird das analytische Hilfsmittel 1 nach der Probenaufnahme zurückgezogen, bis ein elektrischer Kontakt 40 auf den dafür vorgesehenen (nicht dargestellten) Kontaktpads auf dem Testelement 8 aufsitzt. In dieser Position wird eine Messung durchgeführt.

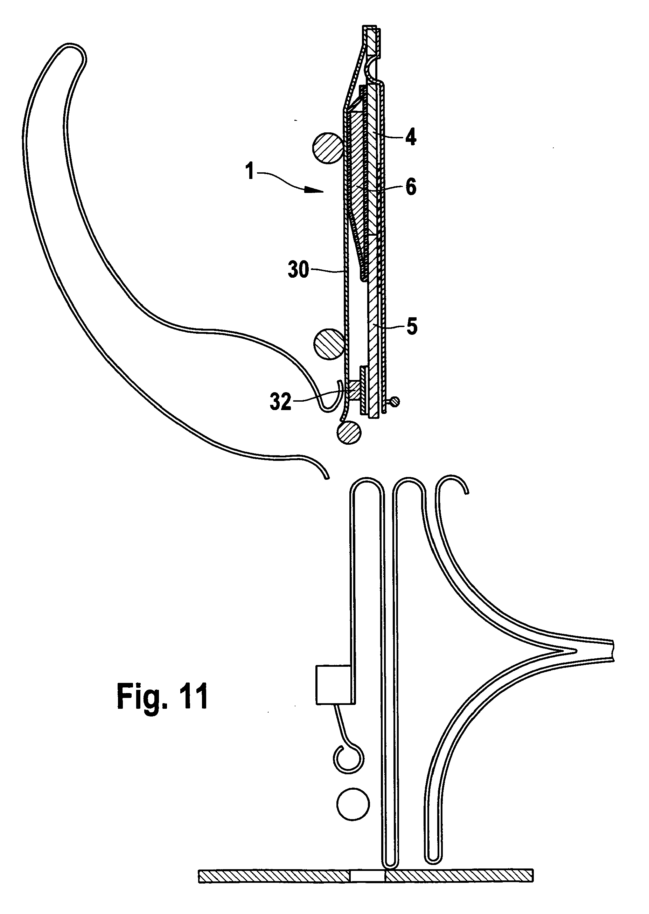

Nach der Messung wird das analytische Hilfsmittel 1 in die Anfangsposition zurückbewegt, wie sie in Figur 11 dargestellt ist. Die Abdeckung 30 wird mittels der Haftzone 32 an dem zweiten Teilkörper 5 befestigt, so dass ein Anwender vor einer zufälligen Berührung der Lanzette 6 und vor einer Kontamination mit Probenresten auf dem gebrauchten analytischen Hilfsmittel 1 geschützt wird.

### Bezugszeichenliste

- 1: analytisches Hilfsmittel
- 2: Grundkörper
- 3: scharnierartige Verbindung
- 4: erster Teilkörper
- 5: zweiter Teilkörper
- 6: Lanzette
- 7: Lanzettenspitze
- 8: Testelement
- 9: Probenaufnahmeort
- 10: Versiegelung
- 11: Ende des zweiten Teilkörpers
- 12: Elektroden
- 13: elektrische Leiterbahnen
- 14: Kontaktpads
- 15: Ausnehmung
- 16: flexibler Streifen
- 17: Testfeld
- 18: Mittel zum Probentransport, Kapillare
- 19: Bewegungsrichtung
- 20: Hautniveau
- 21: Schieber
- 22: erster Umlenker
- 23: zweiter Umlenker
- 24: Finger
- 25: Vorsprung
- 26: Führungselement
- 27: Probenentnahmestelle
- 28: dritter Umlenker
- 29: Blutstropfen
- 30: Abdeckung
- 31: Ende der Abdeckung
- 32: Haftzone
- 33: Halterung
- 34: Mitnehmer
- 35: erste Führungsschiene
- 36: zweite Führungsschiene
- 37: Leitelement
- 38: Kanal
- 39: Öffnung
- 40: elektrischer Kontakt

## Patentansprüche

1. Analytisches Hilfsmittel enthaltend einen Grundkörper (2), eine Lanzette (6) und ein Testelement (8), **dadurch gekennzeichnet**,
i) dass der Grundkörper (2) zwei über eine scharnierartige Verbindung (3) miteinander verbundene Teilkörper (4, 5) umfasst, wobei ein erster Teilkörper (4) die eine Lanzettenspitze (7) aufweisende Lanzette (6) und ein zweiter Teilkörper (5) das ein Testfeld (17) enthaltende Testelement (8) und einen Probenaufnahmeort (9) trägt und
ii) dass die zwei Teilkörper (4, 5) im Wesentlichen in einer gemeinsamen Ebene angeordnet sind und die Lanzette (6) durch eine mit dem ersten Teilkörper (4) verbundene Versiegelung (10) geschützt und von dem Testfeld (17) des Testelements (8) getrennt ist, so lange sich das analytische Hilfsmittel (1) in einem unbenutzten Zustand befindet, wobei die zwei Teilkörper (4, 5) um die scharnierartige Verbindung (3) aus der Ebene gegeneinander verschwenkbar sind und die Versiegelung (10) auftrennbar ist, so dass die Lanzettenspitze (7) zur Benutzung freigegeben wird.

2. Analytisches Hilfsmittel gemäß Anspruch 1, **dadurch gekennzeichnet, dass** die Versiegelung (10) beim Verschwenken der zwei Teilkörper (4, 5) auftrennbar ist.

3. Analytisches Hilfsmittel gemäß einem der Ansprüche 1 oder 2, **dadurch gekennzeichnet, dass** die scharnierartige Verbindung (3) ein flexibler Streifen ist, der die zwei Teilkörper (4, 5) miteinander verbindet.

4. Analytisches Hilfsmittel gemäß einem der Ansprüche 1 bis 3, **dadurch gekennzeichnet, dass** der Probenaufnahmeort (9) im Bereich eines der scharnierartigen Verbindung (3) entgegengesetzten Endes (11) des zweiten Teilkörpers (5) angeordnet ist.

5. Analytisches Hilfsmittel gemäß einem der Ansprüche 1 bis 4, **dadurch gekennzeichnet, dass** die Lanzettenspitze (7) im Bereich der scharnierartigen Verbindung (3) über den ersten Teilkörper (4) hinausragt.

6. Analytisches Hilfsmittel gemäß einem der Ansprüche 1 bis 5, **dadurch gekennzeichnet, dass** die Lanzettenspitze (7) im unbenutzten Zustand des analytischen Hilfsmittels (1) im Wesentlichen parallel zu dem zweiten Teilkörper (5) ausgerichtet ist.

7. Analytisches Hilfsmittel gemäß einem der Ansprüche 1 bis 6, **dadurch gekennzeichnet, dass** die Versiegelung (10) ein taschenartiger Schutz ist, der die Lanzette (6) im unbenutzten Zustand des analytischen Hilfsmittels (1) teilweise oder vollständig umschließt.

8. Analytisches Hilfsmittel gemäß einem der Ansprüche 1 bis 7, **dadurch gekennzeichnet, dass** die Versiegelung (10) bei der Lanzettenspitze (7) fest mit dem zweiten Teilkörper (5) verbunden ist.

9. Analytisches Hilfsmittel gemäß einem der Ansprüche 1 bis 8, **dadurch gekennzeichnet, dass** die scharnierartige Verbindung (3) durch die Versiegelung (10) gebildet wird.

10. Analytisches Hilfsmittel gemäß einem der Ansprüche 1 bis 9, **dadurch gekennzeichnet, dass** das Testelement (8) Mittel zur elektrochemischen oder optischen Analyse einer auf dem Testfeld (17) vorhandenen Probe umfasst.

11. Analytisches Hilfsmittel gemäß einem der Ansprüche 1 bis 10, **gekennzeichnet durch** eine Ausnehmung (15) in dem ersten Teilkörper (4) zum Eingreifen eines Führungselements (26) in einem Analysegerät, **durch** das das analytische Hilfsmittel (1) beim Einstich der Lanzette (6) und bei der Probenaufnahme an eine Probenentnahmestelle (27) herangeführt wird.

12. Analytisches Hilfsmittel gemäß einem der Ansprüche 1 bis 11, **gekennzeichnet durch** eine Abdeckung (30), die auf dem analytischen Hilfsmittel (1) angeordnet ist und die mit dem ersten Teilkörper (4) fest und mit dem zweiten Teilkörper (5) über eine Haftzone (32) lösbar und wieder verbindbar verbunden ist.

13. Analytisches Hilfsmittel gemäß einem der Ansprüche 1 bis 12, **dadurch gekennzeichnet, dass** der zweite Teilkörper (5) ein Mittel zum Probentransport (18) von dem Probenaufnahmeort (9) zu dem Testfeld (17) trägt, das eine Kapillare ist.

14. Magazin, enthaltend mindestens ein in einem Magazin bevorratetes analytisches Hilfsmittel gemäß einem Ansprüche 1 bis 13.

15. Verfahren zur Herstellung von analytischen Hilfsmitteln gemäß einem der Ansprüche 1 bis 13, **dadurch gekennzeichnet, dass** die ersten Teilkörper (4) aus Abschnitten eines ersten Bandes und die zweiten Teilkörper (5) aus Abschnitten eines zweiten Bandes gefertigt werden, wobei die beiden Bänder mit Hilfe von Pilotlöchern relativ zueinander ausgerichtet werden, bevor sie miteinander zu Grundkörpern (2) verbunden werden und die Abschnitte vereinzelt werden.

16. Verfahren gemäß Anspruch 15, **dadurch gekennzeichnet, dass** je eine Lanzette (6) an jeweils einem Abschnitt des ersten Bandes befestigt und mit einer Versiegelung (10) versehen wird und die Lanzetten (6) sterilisiert werden, bevor die beiden Bänder miteinander verbunden werden.

17. Verfahren gemäß Anspruch 16, **dadurch gekennzeichnet, dass** die Versiegelung (10) in einem Vakuum kontrolliert wird.

18. Verfahren zur Analyse einer Probe mit einem analytischen Hilfsmittel gemäß einem der Ansprüche 1 bis 13 mit den Schritten:
A) Einsetzen eines analytischen Hilfsmittels (1) in ein Analysegerät,
B) Verschwenken der zwei Teilkörper (4, 5) gegeneinander aus einer Anfangsposition um einen Winkel α, wobei die Versiegelung (10) aufgetrennt und die Lanzettenspitze (7) über den ersten Teilkörper (4) hinausragend zur Benutzung freigegeben wird,
C) Einstich der Lanzettenspitze (7) in eine Probenentnahmestelle (27),
D) Schwenken der zwei Teilkörper (4, 5) in die Anfangsposition oder in eine andere Probenaufnahmeposition,
E) Aufnahme der Probe an der Probenentnahmestelle (27) auf den Probenaufnahmeort (9) des analytischen Hilfsmittels (1), so dass die Probe auf das Testfeld (17) gelangt und
F) Analyse der Probe auf dem Testfeld (17).

19. Verfahren gemäß Anspruch 18, **dadurch gekennzeichnet, dass** die Lanzettenspitze (7) in Schritt C) aus einer Öffnung (39) des Analysegeräts austritt und der Probenaufnahmeort (9) in Schritt E) aus der Öffnung (39) austritt.

20. Verfahren gemäß einem der Ansprüche 18 oder 19, **dadurch gekennzeichnet, dass** das analytische Hilfsmittel aus einem Magazin in einem Analysegerät bereitgestellt wird.

21. Verfahren gemäß Anspruch 20, **dadurch gekennzeichnet, dass** das analytische Hilfsmittel nach Gebrauch in ein Magazin aufgenommen wird.
